# EUROPEAN PATENT SPECIFICATION

(11) **EP 0 470 959 B1**
(45) Date of publication and mention of the grant of the patent: **30.08.1995**
(21) Application number: 90903630.3
(22) Date of filing: 11.01.1990
(51) Int. Cl.: A61B 17/56, A61L 25/00, A61F 2/46

(54) **DEVICE FOR PREPARING BONE CEMENT**
ANORDNUNG ZUR HERSTELLUNG VON KNOCHENZEMENT
DISPOSITIF POUR LA PREPARATION DE CIMENT POUR OS

(30) Priority: 03.05.1989 SE 8901599
(43) Date of publication of application: 19.02.1992
(73) Proprietor: CEMVAC SYSTEM AKTIEBOLAG, S-311 23 Falkenberg (SE)
(72) Inventor: SMEDS, Staffan, S-582 45 Linköping (SE); JONSSON, Sören, S-582 70 Linköping (SE); THORLING, Jan, S-791 91 Falun (SE); NILSSON, Thomas, S-311 45 Falkenberg (SE)
(74) Representative: Willquist, Bo
(86) International application number: PCT/SE90/00018
(87) International publication number: WO 90/13264

(56) References cited:
- WO-A-87/05492
- WO-A-88/03811
- DK-B- 0 123 745
- FR-A- 2 286 657
- SE-B- 0 447 785
- US-A- 4 208 133

## Description

The present invention relates to a method and an arrangement for the manufacture of bone cement by mixing together its constituent components according to the preambles of claims 1 and 4 respectively.

Bone cement is used in hip-joint operations, for example. When manufacturing bone cement, for which purpose a component in the form of a powder and a liquid component require to be mixed, it is important that the mixture should be as homogeneous as possible, and that gases which occur during the mixing procedure are removed to the greatest extent possible. Any lack of homogeneity and gas inclusions have the effect of reducing the strength of the bone cement, which can result in a shorter service life for the implanted prosthesis. Furthermore, the gases which occur during manufacture are unhealthy, for which reason steps must be taken to ensure that they do not escape into the operating theatre.

Swedish document SE, B, 447 785 (& US-A-4 758 096) discloses an arrangement for the manufacture of bone cement comprising a mixing cylinder which is placed in a vacuum chamber in order to create vacuum in the mixing cylinder during mixing to ensure that no bubbles are formed in the bone cement. The components are loaded into the mixing cylinder prior to the mixing process. Once mixing is finished, the cylinder is removed from the vacuum chamber, a discharge pipe is connected to the cylinder and the mixed bone cement is manually extruded from the mixing cylinder.

Patent application WO, A1, 8705492 relates to a device and a process for mixing bone cement and filling an application holder with said cement. The device has a mixer and a sealing ring which can be coupled to the application holder. The application holder is located in the mixing receptacle and is movable in relation to the latter. In one embodiment, the mixer contains the substances to be mixed in separate chambers and as a result of relative movement between the application holder and the mixing receptacle, the substances are mixed and filled into the application holder.

In US-A-4 208 133, upon which the preambles of claims 1 and 4 are based, an injection cartridge with at least two separate chambers is disclosed, with each chamber holding a component which can be mixed with the other component in the cartridge prior to use. Such an injection cartridge is primarily intended for use in the building industry for the anchoring of plugs, tie bolts of the like. A disadvantage with such an arrangement is that the quantities of the components and the mixing ratio cannot be varied since the components are contained in sealed chambers.

An object of the present invention is to make available a method and a preparation arrangement for bone cement, which arrangement is intended to be disposable and capable of being manufactured efficiently and at a low cost, and which also contributes to meeting the requirements stipulated above in respect of the prepared bone cement, whilst being more flexible than the arrangement disclosed in US-A-4 208 133.

This object is met in accordance with the present invention by a method according to claim 1 and an arrangement according to claim 4.

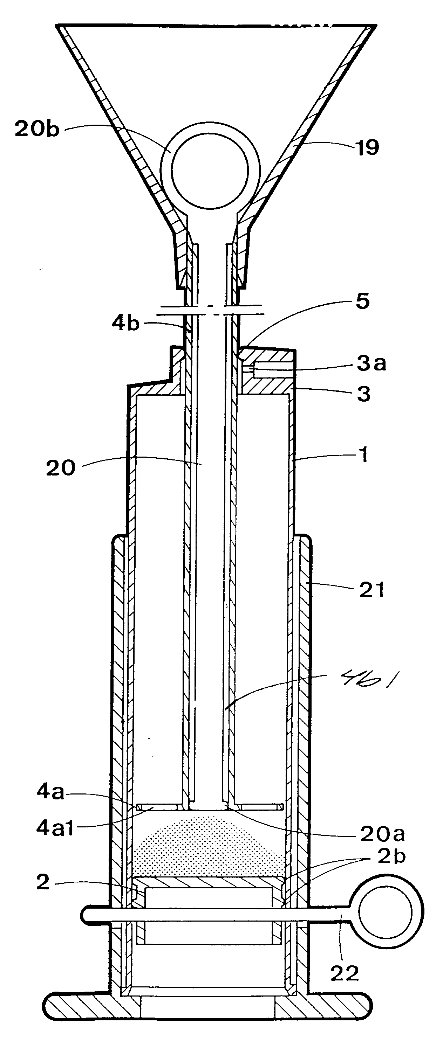

Additional advantages of the invention can be appreciated from the accompanying respective dependent claims and from the following description with reference to the accompanying drawing which illustrates schematically a longitudinal section through an embodiment of the preparation arrangement in accordance with the invention.

The designation 1 is used in the drawing for a mixing cylinder, and 2 for a bottom which is inserted in a sealing fashion into one end of the mixing cylinder 1. The mixing cylinder 1 is preferably made from a transparent material, and the bottom 2 is made from a rubber-like elastic material.

The preparation arrangement in accordance with the invention also includes a lid 3 with an agitator 4 capable of axial movement therein. This comprises a piston-like agitator disc 4a and an agitator rod 1b supported in the lid 3 in such a way that it is free to slide. The mixing cylinder 1 and lid 3 are manufactured in a single piece and the lid 3 has an evacuation channel 3a and a seal 5 against the agitator rod 4b. The bottom 2 is formed as an outwardly open cup with annular flange-like seals 2b in contact with the inner wall of the mixing cylinder 1.

The agitator disc 4a is perforated by channels 4a1, through which the bone cement components are caused to flow during agitation in order to make this as effective as possible. The agitator rod 4b exhibits in accordance with the invention an axial channel 4b1. With the help of the agitator rod 4b, the agitator disc 4a is caused to move up and down inside the mixing cylinder 1. Since the cylinder wall is transparent, a person skilled in the art can easily decide when the preparation of the bone cement is complete.

A funnel 19 is removably attached to the outer end of the agitator rod 4b and a rod 20 is introduced into the axial channel 4b1 of the agitator rod 4b, together with a tubular holder 21 into which the mixing cylinder 1 is introduced. The narrow opening of the funnel 19 is in close contact with the agitator rod 4b, and its purpose is to facilitate the introduction of the bone cement components into the mixing cylinder 1, for which purpose the rod 20 must have been pulled from the channel 4b1. The rod 20 is of a length such that, when introduced fully into the channel 4b1, it reaches into the vicinity of the agitator disc 4a and is in this area executed with a seal 20a in contact with the inner wall of the channel 4b. The rod 20 with the seal 20a sealingly closes one end of the channel 4b1. At its opposite end the rod 20 is executed with a draw ring 20b or similar. The mixing cylinder 1 and the piston 2 are fixed relative to the holder 21 by means of a removable cotter pin 22.

The preparation arrangement is used as follows: The rod 20 is first removed so that the bone cement components can be introduced into the mixing cylinder 1 via the channel 4b1. The funnel 19 is then removed, and the rod 20 is introduced into the channel 4b1. The bone cement components are now mixed together; any gases which occur are removed via the channel 3a. During the mixing procedure the arrangement is placed in the holder 21. Once the mixing procedure is complete, the cotter pin 22 is withdrawn from its position, thereby enabling the preparation arrangement to be removed freely from its holder 21. The preparation arrangement is now placed in a syringe mechanism of the kind which is customarily used for the extrusion of sealing compound or similar in the form of a bead. Once the cotter pin 22 has been removed, the piston 2 can be displaced axially under the effect of the compression piston 15. Once the rod 20 has been removed from the channel 4b1, bone cement can be forced out through it for the intended use.

## Claims

1. A method for manufacturing bone cement by mixing together its constituent components, said method comprising the steps of:
providing a mixing arrangement comprising a mixing cylinder (1) in the form of a tube with a bottom (2) and a lid (3), and an axially movable agitator (4) inside the mixing cylinder (1), which agitator consists of an agitator disc (4a) which is perforated by channels (4a1) through which the components of the bone cement are caused to flow during the mixing procedure, and an agitator rod (4b) that is operatively connected to the agitator disc and supported in the lid (3) in such a manner that it is free to slide, said agitator rod (4b) presenting an axial channel (4b1) having an opening at the end of the agitator rod attached to the agitator disc (4a), which opening is closed off during mixing of the constituent components and opened to allow discharge of the bone cement, the bottom (2) being so arranged that, under the effect of an axial force, it is displaceable like a piston axially in the direction of the lid (3);
providing the mixing arrangement with the constituent components;
mixing together the constituent components by sliding the agitator (4) within the mixing cylinder (1);
opening the opening to the axial channel (4b1) in said agitator rod (4b), and
displacing the bottom (2) towards the lid (3) to thereby discharge the bone cement from the mixing cylinder (1),
**characterized by** providing the mixing arrangement with the constituent components by introducing the components into the mixing cylinder (1) via said axial channel (4b1) in the agitator rod (4b).

2. The method as claimed in claim 1, **characterized by** connecting a vacuum source to cooperate with the interior of the mixing cylinder (1).

3. The method as claimed in claim 1 or claim 2, **characterized by** introducing the components into the mixing cylinder (1) via a funnel (19) removably attached to the outer end of the agitator rod (4b).

4. An arrangement for manufacturing bone cement by mixing together its constituent components, comprising a mixing cylinder (1) in the form of a tube with a bottom (2) and a lid (3), and an axially movable agitator (4) inside the mixing cylinder (1), which agitator consists of an agitator disc (4a) which is perforated by channels (4a1) through which the components of the bone cement are caused to flow during the mixing procedure, and an agitator rod (4b) that is operatively connected to the agitator disc and supported in the lid (3) in such a manner that it is free to slide, said agitator rod (4b) presenting an axial channel (4b1) having an opening at the end of the agitator rod attached to the agitator disc (4a), which opening is closed off during mixing of the constituent components and opened to allow discharge of the bone cement, the bottom (2) being so arranged that, under the effect of an axial force, it is displaceable like a piston axially in the direction of the lid (3), thereby causing the bone cement to flow out through the channel (4b1), **characterized in that** said mixing vessel (1) is provided with an evacuation channel (3a) to which a vacuum source is connectable, and in that said arrangement further comprises a funnel (19) removably attached to the outer end of the agitator rod (4b) to facilitate the introduction of the bone cement components into the mixing cylinder (1).

5. The arrangement as claimed in claim 4, **characterized in that** said evacuation channel (3a) is provided in the lid (3) of the mixing cylinder (1).

## Patentansprüche

1. Verfahren zum Herstellen von Knochenzement durch Durchmischen von dessen Komponenten, mit den folgenden Verfahrensschritten:
es wird eine Mischanordnung vorgesehen, umfassend einen Mischzylinder (1) in Gestalt einer Hülse mit einem Boden (2) und einem Deckel (3) sowie einem axial beweglichen Rührer (4), der sich innerhalb des Mischzylinders (1) befindet und aus einer Rührerscheibe (4a) besteht, die von Kanälen (4a1) durchzogen ist, durch welche die Komponenten des Knochenzements während des Mischvorganges strömen gelassen werden, ferner einer Rührerstange (4b), die mit der Rührerscheibe in Wirkverbindung steht und derart vom Deckel (3) getragen ist, daß sie frei gleiten kann, wobei die Rührerstange (4b) einen Axialkanal (4b1) aufweist, der an jenem Ende der Rührerstange, das an der Rührerscheibe (4a) befestigt ist, eine Öffnung aufweist, die ihrerseits während des Mischens der Komponenten abgeschlossen ist und zum Zwecke der Abgabe des Knochenzements geöffnet ist, wobei der Boden (2) derart angeordnet ist, daß er unter der Wirkung einer Axialkraft gleich einem Kolben axial in Richtung des Deckels (3) bewegbar ist; es wird die Mischanordnung mit den Komponenten versorgt;
die Komponenten werden durch Verschieben des Rührers (4) innerhalb des Mischzylinders (1) miteinander durchmischt;
die Öffnung zum Axialkanal (4b1) in der Rührerstange (4b) wird geöffnet, und
der Boden (2) wird gegen den Deckel (3) hin verfahrbar, um hierdurch den Knochenzement aus dem Mischzylinder (1) abzugeben,
dadurch gekennzeichnet, daß bei der Mischanordnung mit den Komponenten diese Komponenten in den Mischzylinder (1) über den Axialkanal (4b1) in der Rührerstange (4b) eingeführt werden.

2. Verfahren nach Anspruch 1, dadurch gekennzeichnet, daß eine Vakuumquelle angeschlossen wird, um mit dem Innenraum des Mischzylinders (1) zusammenzuarbeiten.

3. Verfahren nach Anspruch 1 oder 2, dadurch gekennzeichnet, daß die Komponenten in den Mischzylinder (1) über einen Trichter (19) eingeführt werden, der entfernbar am Außenende der Rührerstange (4b) befestigt ist.

4. Anordnung zum Herstellen von Knochenzement durch Durchmischen von dessen Komponenten, mit einem Mischzylinder (1) in Gestalt einer Hülse mit einem Boden (2) und einem Deckel (3), ferner mit einem axial beweglichen Rührer (4), der sich innerhalb des Mischzylinders (1) befindet und aus einer Rührerscheibe (4a) besteht, die von Kanälen (4a1) durchzogen ist, durch welche die Komponenten des Knochenzementes während des Mischvorganges strömen gelassen werden, und einer Rührerstange (4b), die mit der Rührerscheibe in Wirkverbindung steht und vom Deckel (3) derart getragen ist, daß sie frei gleiten kann, wobei die Rührerstange (4b) einen Axialkanal (4b1) aufweist, mit einer an demjenigen Ende der Rührerstange, an dem die Rührerscheibe (4a) befestigt ist, vorgesehenen Öffnung, die während des Mischens der Komponenten abgesperrt ist, und die zum Ablassen des Knochenzementes geöffnet wird, wobei der Boden (2) derart angeordnet ist, daß er unter der Einwirkung einer Axialkraft gleich einem Kolben in Richtung des Deckels (3) verfahrbar ist, um den Knochenzement aus Kanal (4b1) austreten zu lassen, dadurch gekennzeichnet, daß der Mischzylinder (1) mit einem Entlüftungskanal (3a) versehen ist, an welchen eine Vakuumquelle anschließbar ist, und daß die Anordnung ferner einen Trichter (19) aufweist, der abnehmbar am Außenende der Rührerstange (4b) befestigt ist, um das Einführen der Knochenzementkomponenten in den Mischzylinder (1) zu erleichtern.

5. Anordnung nach Anspruch 4, dadurch gekennzeichnet, daß der Entlüftungskanal (3a) im Deckel (3) des Mischzylinders (1) vorgesehen ist.

## Revendications

1. Procédé de fabrication de ciment pour os par un mélange intime de ses constituants, ledit procédé comprenant les étapes consistant à :
prévoir un dispositif de malaxage comprenant un cylindre malaxeur (1) sous la forme d'un tube ayant un fond (2) et un couvercle (3), et un agitateur (4) axialement mobile à l'intérieur du cylindre malaxeur (1), lequel agitateur se compose d'un disque d'agitateur (4a) qui est perforé par des passages (4a1) par lesquels les constituants du ciment pour os sont amenés à s'écouler pendant l'opération de mélange, et une tige d'agitateur (4b) qui est reliée au disque d'agitateur et soutenue dans le couvercle (3) de manière à pouvoir coulisser librement, ladite tige d'agitateur (4b) présentant un passage axial (4b1) ayant une ouverture au niveau de l'extrémité de la tige d'agitateur, fixée au disque d'agitateur (4a), laquelle ouverture est obturée pendant le malaxage des constituants, et ouverte pour permettre la sortie du ciment pour os, le fond (2) étant agencé de telle manière que, sous l'effet d'une force axiale, il puisse se déplacer à la manière d'un piston, axialement en direction du couvercle (3) ;
remplir le dispositif de malaxage des constituants ;
mélanger intimement les constituants en faisant coulisser l'agitateur (4) à l'intérieur du cylindre malaxeur (1) ;
ouvrir l'ouverture donnant accès au passage axial (4b1) dans ladite tige d'agitateur (4b), et
déplacer le fond (2) en direction du couvercle (3) pour ainsi faire sortir le ciment pour os hors du cylindre malaxeur (1),
**caractérisé en ce que** les constituants sont amenés au dispositif de malaxage en introduisant les constituants dans le cylindre malaxeur (1) par l'intermédiaire dudit passage central (4b1) présent dans la tige d'agitateur (4b).

2. Procédé selon la revendication 1, **caractérisé par** le raccordement d'une source de dépression pour coopérer avec l'intérieur du cylindre malaxeur (1).

3. Procédé selon la revendication 1 ou la revendication 2, **caractérisé par** l'introduction des constituants dans le cylindre malaxeur (1) au moyen d'un entonnoir (19), fixé, de manière détachable, à l'extrémité extérieure de la tige d'agitateur (4b).

4. Dispositif pour la fabrication de ciment pour os par un mélange intime des constituants de celui-ci, comprenant un cylindre malaxeur (1) sous la forme d'un tube ayant un fond (2) et un couvercle (3), et un agitateur (4) axialement mobile à l'intérieur du cylindre malaxeur (1), lequel agitateur se compose d'un disque d'agitateur (4a) qui est perforé par des passages (4a1) par lesquels les constituants du ciment pour os sont amenés à s'écouler pendant l'opération de mélange, et une tige d'agitateur (4b) qui est reliée au disque d'agitateur et soutenue dans le couvercle (3) de manière à pouvoir coulisser librement, ladite tige d'agitateur (4b) présentant un passage axial (4b1) ayant une ouverture au niveau de l'extrémité de la tige d'agitateur, fixée au disque d'agitateur (4a), laquelle ouverture est obturée pendant le malaxage des constituants, et ouverte pour permettre la sortie du ciment pour os, le fond (2) étant agencé de telle manière que, sous l'effet d'une force axiale, il puisse se déplacer à la manière d'un piston, axialement en direction du couvercle (3), pour ainsi provoquer la sortie du ciment pour os par l'intermédiaire du passage (4b1), **caractérisé en ce que** ledit récipient de malaxage (1) est pourvu d'un conduit d'évacuation (3a) auquel peut être raccordée une source de dépression, et en ce que ledit dispositif comprend en outre un entonnoir (19), fixé de manière détachable à l'extrémité extérieure de la tige d'agitateur (4b) pour faciliter l'introduction des constituants du ciment pour os dans le cylindre malaxeur (1).

5. Dispositif selon la revendication 4, **caractérisé en ce que** ledit conduit d'évacuation (3a) est prévu dans le couvercle (3) du cylindre malaxeur (1).
